# EUROPEAN PATENT APPLICATION

(11) **EP 2 085 077 A1**
(43) Date of publication of application: **05.08.2009**
(21) Application number: 08001683.5
(22) Date of filing: 30.01.2008
(51) Int. Cl.: A61K 9/16

(54) **Coating compositions for controlled relaease formulations**

(71) Applicant: Südzucker Aktiengesellschaft Mannheim/Ochsenfurt, 68165 Mannheim (DE)
(72) Inventor: Kimmel, Michael, DE-68239 Mannheim (DE); Luhn, Oliver, DE-67134 Birkenheide (DE); Antal, Istvan Dr., HU-1165 Budapest (HU)
(74) Representative: Schwahn, Hartmut

(57) **Abstract**

The invention concerns coating compositions for controlled, sustained or extended release in particular from particulate drug formulations, controlled release drug formulations, and methods and means to produce such formulations, including substrates coated with such compositions, films made from the composition and methods for making and using such compositions.

## Description

The invention concerns coating compositions for controlled, sustained or extended release in particular from particulate drug formulations, controlled release drug formulations, and methods and means to produce such formulations, including substrates coated with such compositions, films made from the composition and methods for making and using such compositions. The invention particularly concerns processing and formulation approaches for improving the solubility of poorly soluble drugs in controlled, sustained or extended release solid oral dosage forms and multiple-unit controlled or extended release dosage forms - such as "retard" formulations.

### Background art

The coating of starter pellets or tablets for the purpose of modifying the drug release profile, for example enteric coatings, sustained release coatings, osmotic pumps, etc. are known in the art. Currently film-coatings are preferred which are defined as thin and uniform polymer based coats of out 20 to 100 µm of thickness and are applied to the surface of substrates such as tablets, granules, powders, capsules, multiparticulates or pellets. Compared to sugar coating, film-coating provides more flexibility with the ability to coat a variety of substrates, other than just compressed tablets or pellets. Film-coating is accomplished mainly by using a fluidised bed coating column preferably equipped with a bottom spray appliance (e.g. Wurster Coating). A coating composition is dissolved or dispersed in an organic or water solvent system and is sprayed on the fluidized substrates.

The polymer films are primarily applied to medicinal drug preparations in order to control the release rate of drugs (API = the active ingredient of the drug products). Conventionally, a polymeric substance which is the film base composition is dissolved in an organic solvent and is supplied for the process of film coating. Due to environmental and safety concerns, aqueous film coating compositions have come into use in order to avoid the use of organic solvents. Typical film-coating formulation components are polymers which are flexible linear macromolecules. Water-insoluble polymers in aqueous dispersion are particularly useful for modified (sustained, extended) release coating applications. Such polymer dispersions are classified into two types: true latexes and pseudo latexes. True latexes which are a very fine dispersions of the polymer in an aqueous phase with a particle size range of 10 to 1000 nm. True latexes are produced by emulsion polymerisation of monomer, initiator and catalyst activated polymers. Eudragit (Rhöm Pharma, DE) is a common polymer for true latex dispersions. In the coating process the polymer particles fuse together to form a discrete film. The glas transition temperature of the polymer can be lowered by the incorporation of plasticisers.

The release profile of such coated formulations is influenced and controlled mainly by the use of agents that modify the permeability of the film coat. For example, permeability is increased by incorporating swellable polymers into the coating. Dependent on the functional groups present the swellable polymers the increase of permeability can be pH dependent. The pH dependency is of particular importance in enteric coatings for the controlled or sustained release of drugs in certain sections of the intestine.

Another group of compounds serve to form pores within the rather water-impermeable film coat and by that facilitate permeation of water and drugs through the insoluble coating.

Despite the present improvements in controlled release coatings, it still is a challenge to provide controlled or sustained release formulations for compounds or APIs that exhibit rather low solubility in water. For example, diclofenac sodium salt (diclofenac-Na) has a solubility as low as 2.4 mg/mL (at 25° C). Diclofenac-Na is found to be released very poorly from common controlled release formulations, which are based on film coated pellets with a drug-layered inert core, even if swellable polymers or common pore forming components like soluble monosaccharides are incorporated in the coating composition.

The technical problem of the present invention is to provide film coating compositions for controlled release drug formulations, in particular for particulate or multi-particulate formulations that offer greater variety and flexibility in the release profile to be obtainable and in the compound / API to be usable. In particular, such coating compositions should be usable in completely sugar-free formulations. Further, such coating compositions should be usable for drug formulations that comprise compounds / APIs with low solubility in water.

The technical problem is primarily solved by the provision of a coating composition for a controlled or sustained release drug formulation mainly as characterized in claim 1. According to the invention, the coating composition comprises at least:
- a film-forming component which comprises one or more of polymeric binders; and
- a release controlling agent which is selected from disaccharide alcohols and disaccharide alcohol compositions.

The coating can be performed by art recognized techniques.

The invention is mainly based on the surprising finding that disaccharide alcohols and disaccharide alcohol compositions advantageously control drug release through rather impermeable polymeric films like, for example, Eudragit® RS (Rhöm Pharma, DE). In comparison to pore forming agents known in the art, like monosaccharides, the controlled release agent of the invention particularly confers improved release profiles for drugs / API with low solubility. The effect will be most prominent in particular if in the drug formulation, in particular a particulate drug formulation, sugar-free inert cores such as microcrystalline cellulose MCC, for example, Ethispheres® (NP Pharm, FR), are employed. Most surprisingly, the coatings according to the invention increases drug release in acidic media (e.g. pH 4.5).

Without wishing to be bound, the disaccharide alcohols and disaccharide alcohol compositions mainly serve to form pores in the polymeric film and, at the same time, may directly interact with the film-forming polymer.

A preferred disaccharide alcohol composition, Isomalt (also known as galenIQ® from Palatinit, DE; Isomalt Ph. Eur.; B.P.; U.S.P. 29-NF24), a disaccharide alcohol composition comprising 6-O-α-D- glucopyranosyl-D-sorbitol (1,6-GPS) and 1-O-α-D-glucopyranosyl-D-mannitol (1,1-GPM) as main constituents, for example in a racemic ratio. Methods to produce Isomalt are widely known in the art. EP 0 847 242 A1 concerns the production of 1,6 GPS-enriched Isomalt compositions and is fully incorporated hereinwith by reference. The disaccharide alcohols and alcohol compositions of the invention support the controlled release of drugs with a rather low solubility, in particular a solubility in water (at 25°C) of 50 mg/mL or less, or even of 10 mg/mL or less, more particularly of 5 mg/mL or less. The coating compositions of the invention advantageously allows the provision of sustained release drug formulations of AlPs which exhibit a low solublility in water, e.g. diclofenac-Na.

The coating composition of the invention may be used to coat various cores or substrates containing the active ingredient such as tablets, spheroids (or beads), microspheres, seeds, pellets, or other multi-particulate systems, in order to obtain a desired controlled release of the active agent. Granules, spheroids, pellets, and the like can be presented in a capsule or in another suitable dosage form. The tablets can be any suitable shape, such as round, oval, biconcave, hemispherical or any polygonal shape, such as square, rectangular, pentagonal and the like.

The invention also provides a coated drug formulation in particular a particulate formulation that is coated with the controlled release coating composition of the invention; i.e. the coat of said drug formulation comprises the controlled release coating of the invention.

In a preferred embodiment the drug formulation is comprised of (a) an inert inner core, (b) at least one intermediate drug layer that is containing the drug or drug composition, and (c) the controlled release coating of the invention.

In another preferred embodiment the particulate drug formulation is comprised of (a) an inner core that is containing the drug or drug composition and (b) the controlled release coating of invention.

Another preferred embodiment of the invention is a preferably multi-particulate, formulation usable in medical, nutritional and/or cosmetic applications. The multiparticulate formulation of the invention comprises at least two i.e. a multitude of the particulate formulations according to the invention. The multiparticulate formulation is preferably in the form of a capsule or compacted tablet of agglomerated coated particles.

In this invention, the film coated particles can be administered as is, or can be used mixed with other drug preparations, or can be mixed with other vehicles and drugs or particles that contain drugs or particles that have been subjected to film coating, after which they can be finished as tablets or pills. In addition to medicinal or veterinary uses, they can also be used for agricultural chemicals, fertilizers, foods, cosmetics or industrial applications.

Another embodiment of the invention is a method for producing a preferably particulate, formulation with controlled release property. The method or process comprises at least the following steps: (a) providing a starter pellet as inert inner core; (b) layering the starter pellet with a layer comprising drug or drug composition or any other type of active principle for medical, nutritional or cosmetic uses; and, preferably immediately after, (c) coating the layered pellet with the coating composition of the invention.

Yet another embodiment of the invention is a method of use or the use of disaccharide alcohol or disaccharide alcohol composition as release controlling agent in or for controlled release coating compositions that are preferably apt for particulate formulations for medical, nutritional or cosmetic uses.

### Brief description of the drawings

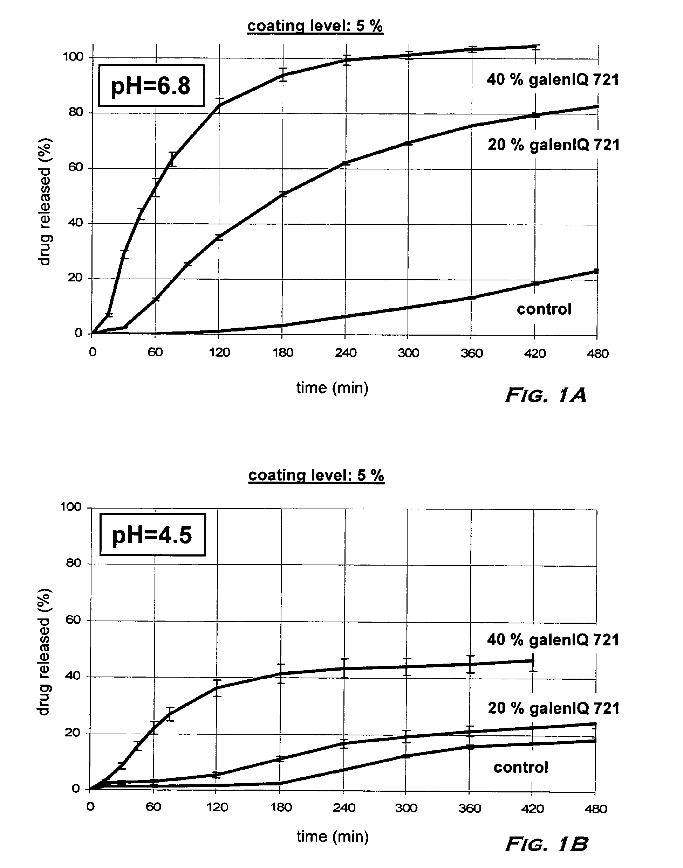

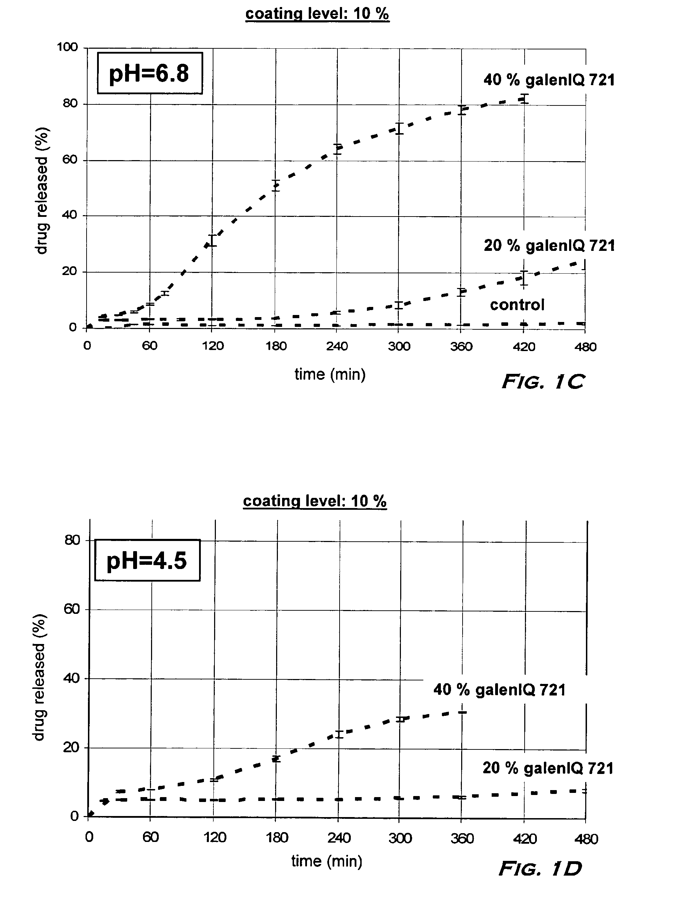

Figures 1A to D illustrate the results of U.S.P. No. 1 dissolution test: Release profiles for diclofenac-Na; inert MCC cores are layered with diclofenac-Na and coated with different coating compositions: Eudragit® RS 30 D (control), Eudragit® RS 30 D + 20 % galenIQ® 721 (according to invention), Eudragit® RS 30 D + 40 % galenIQ® 721 (according to invention).

### Detailed description of the invention

### Coating compositions

In a preferred embodiment, the coating composition comprises disaccharide alcohol or disaccharide alcohol composition in an amount from 1 to 20 % by weight, preferably from 2 to 10 % by weight of dry matter of the coating composition. It is preferred that the coating composition comprises the disaccharide alcohol or disaccharide alcohol composition in an amount of from 5 to 50 % by weight, preferably from 15 to 45 % by weight of the film-forming component dry matter.

The disaccharide alcohol or disaccharide alcohol composition is selected from at least one of: 1,6-GPS, 1,1-GPM, maltitol, lactitol. The coating composition of the invention thus preferably is sugar-free.

In a preferred embodiment, the disaccharide alcohol composition of the coating comprises 1,6-GPS from 1 to 99 % by weight and 1,1-GPM from 99 to 1 % by weight. In a preferred variant the disaccharide alcohol composition of the coating comprises Isomalt, comprising a racemic mixture of 1,6-GPS and 1,1-GPM, that is 1,6-GPS from more than 47 to less than 53 % by weight and 1,1-GPM from less than 53 to more than 47 % by weight, respectively. In another preferred variant the disaccharide alcohol composition of the coating comprises an Isomalt composition which is enriched in 1,6-GPS, that is 1,6-GPS from 53 to 99 % by weight and 1,1-GPM from 47 to 1 % by weight, respectively. The disaccharide alcohol composition may further comprise 1,1-GPS. A most preferred variant is an Isomalt-composition where 1,6-GPS and 1,1-GPM are present in a ratio of about 3:1. Such preferred variants are known, for example, as galenIQ® (Palatinit, DE). In another preferred embodiment the disaccharide alcohol of the coating is maltitol. In yet another preferred embodiment the disaccharide alcohol of the coating is lactitol.

The disaccharide alcohol or composition thereof is preferably provided as agglomerated particles to be added to the coating composition. A particle size distribution in the range of about 90-220-360 (d10-d50-d90-quantiles, as measured by mechanical sieve shaker) is preferred. This variant is known, for example, as galenIQ® 721 (Palatinit, DE). In an alternative approach, milled crystals are utilized, having a particle size distribution in the range of about 9-22-41 (d10-d50-d90-quantiles, as measured by laser diffraction). Such a variant is known, for example, as galenIQ® 800 (Palatinit, DE).

In the coating composition, the film-forming polymer is preferably present in the form of a latex dispersion. In an alternative variant the film-forming polymer is present in the form of a pseudolatex dispersion. The dispersions are preferably prepared by emulsion polymerization. The dispersions are preferably fully aqueous dispersions and most preferred no organic solvents are present in the composition to be applied on the substrates. Upon coating the polymer particles of the dispersion coalesce and finally fuse together to form a discrete film.

A "latex dispersion" is a synthetic resin dispersion of solid particles of a water insoluble polymer in water. The polymer particles preferably exhibit an average particle size of 0.2 to 3 µm. As aqueous synthetic resin dispersion for the coating composition according to the present invention, any of the pharmacologically compatible, water insoluble polymeric film formers described herein can be used. Latex dispersions include, but are not limited to polyvinyl esters, polyvinyl acetals, polyacrylic acid esters, cellulose ethers, cellulose esters, butadiene styrene copolymers, methacrylic and acrylate polymers, co-polymers thereof, ethyl cellulose, polyvinylacetate, and cellulose acetate, and the like. Preferred are polymethacrylate polymers, in particular

Eudragit® RS (Röhm Pharma, DE). The film former is present in the coating composition in amounts of 50% or more by weight, 60% or more by weight, 65% or more by weight, 70% or more by weight, 75% or more by weight, 85% or more by weight, or 90% or more by weight, all on a dry basis.

In general, the barrier properties of the pseudolatex or latex film depend on the film thickness or loading, the amount and type of plasticizer, and the degree of coalescence of the pseudolatex or latex particles in the film, which is, in turn, time, temperature and humidity dependent. Optimisation and scale-up of aqueous pseudolatex or latex film coating processes can be complex. Partially coalesced films will give faster release rates but the release rates will not be stable with time as coalescence may still proceed, albeit slowly, under normal storage conditions. If the fully coalesced barrier properties are excessive then the thickness or the loading can be reduced.

The coating composition thus preferably further comprises at least one plasticizer. A plasticizer is preferably used to alter the physical properties of the polymeric binder. Plasticizers may be used to influence on mechanical properties of the polymer film and/or to lower the glass transition temperature of the polymer to allow a more feasible coating process. In a variant of the invention, the plasticizers are used to influence the permeability characteristics of the film, especially to water vapor. as well as . Types of plasticizers that may be used in the coating composition according to the invention are: (a) polyols: glycerin, propylene glycol (PG), polyethylene glycol (PEG in particular 200-6000 grades); (b) water insoluble organic esters: diethyl phthalate (DEP), dibutyl phthalate (DBP), dibutyl sebacate (DBS), dibutyl adipate (DBA), acetyltriethyl citrate (ATEC), acetyltributyl citrate (ATBC), tributyl citrate (TBC); (c) water miscible organic esters: triethyl citrate (TEC), triacetin (glyceryl triacetate; TA); (d) oils/glycerides: castor oil, distilled acetylated monoglycerides (AMG), fractionated coconut oil. Among those, triethyl citrate (TEC) is preferred.

It will be understood that the plasticizer used may be largely dictated by the polymer used in the coating composition. According to the invention the plasticizer is used at concentrations preferably from 10 to 40 %, more preferred by 15 to 35%, based on polymer weight. The plasticizer may be present in the coating in amounts ranging from about 1% to about 25% by weight and more preferably from about 5 to about 15% by weight based on the dry weight of the coating.

In preferred embodiments of the coating composition one or more further excipients are present: Such excipients include, but are not limited to: pigments or opacifiers, vehicles and solvents, flavors, adhesion enhancers, surfactants or dissolution enhancers, and anti-tacking agents/glidants. It is within the purview of one of ordinary skill in the art to determine how much excipient is to be added and the objective that he wishes to accomplish by adding the same.

Pigments or opacifiers are used in film coating to enable product identification (color, appearance) and/or light protection. Pigments or opacifiers may also be used to modify the gas permeability of the film coat. Suitable ingredients for providing color to the formulation include titanium dioxide and color pigments, such as iron oxide pigments, FD & C Yellow No. 6, FD & C Red No. 2, FD & C Blue No. 2, food lakes and the like. If present, they are present in amounts ranging from about 0.1 % to about 20% by dry weight of the coating and more preferably less than about 3% by dry weight.

Solvents and vehicles are preferably used to carry the coating materials to the surface of the product core. They include water, alcohols, ketones, esters, and chlorinated hydrocarbons. The type of vehicle or solvent used during the coating process can be used to classify the type of film coating into an organic film coating (non-aqueous film coating), or an aqueous film coating. The solvent interacts with the selected polymer in order to allow both film adhesion and mechanical strength to be optimized.

Flavors may be included in some oral formulations to make them more palatable or to support product recognition.

Adhesion enhancers are preferably selected from saccharides such as polydextrose, maltodextrin, and lactose and the like. They are preferably used to increase adhesion, for example of cellulosic polymers, to the substrate as well as to improve stability towards light, for example with rather unstable colors.

Surfactants or dissolution enhancers are preferably selected from xanthan gum and the like.

Anti-tacking agents, i.e. glidants or lubricants, are preferably present in the coating composition. They are selected, but are not limited to talc, aluminium, calcium and magnesium stearate, kaolin, colloidal silicon dioxide, glyceryl monostearate. Typically they are used to reduce sticking during film formation. A most preferred glidant is talc. If present, the glidant is present in amounts ranging from about 0.1% to about 10% by dry weight of the coating.

Additionally, the coating compostion may contain preservatives (i.e. sorbic acid), antifoaming agents (i.e. dimethylpolysiloxane), further stabilizing agents, and/or waxes.

### Core composition

In a preferred embodiment a substrate is coated with the coating composition of the invention. The substrate is selected from pellets, tablets, soft capsules, hard capsules, powders, granules, beads, multiparticulates, films and film enrobed dosage forms. The coated substrate preferably is a drug matrix or a core coating with a drug layer. It is preferred that the coated substrate, e.g. coated tablet, pellet or multiparticular formulation is completely free of sugars. In such an embodiment the substrate, inert core or starter pellet is sugar-free and is comprised of microcrystalline cellulose (MCC, such as described hereinbelow) or similar components or bulking substances. In an alternative approach the substrate, inert core or starter pellet is sugar-free and is comprised of saccharide alcohol or saccharide alcohol compositions.

Pellets comprising microcrystalline cellulose (MCC) as a base are preferably used as cores to be coated in the manufacture of dry pharmaceutical forms. These pellets enable greater accuracy and consistency in drug layering and coating. Such pellets are known, for example, as Ethispheres® (NP Pharm, FR). MCC's high physicochemical inertness improves the stability of the finished drug formulations. MCC's high porosity increases the quantity of API absorbed in the drug layering process. Thus, the use of MCC-based pellets or substrates is desirable. The present invention greatly improves the usability of MCC pellets in controlled release formulations.

Also preferred is a disaccharide alcohol or disaccharide alcohol composition, selected from at least one of 1,6-GPS, 1,1-GPM, maltitol, lactitol and mixtures thereof. Among those, disaccharide alcohol compositions with the main constituents 1,6-GPS and 1,1-GPM are most preferred. In a preferred variant of the embodiment the disaccharide alcohol composition of the substrate comprises 1,6-GPS from more than 47 to less than 53 % by weight and 1,1-GPM from less than 53 to more than 47 % by weight, respectively. Preferred are racemic mixtures where 1,6-GPS and 1,1-GPM are present in a ratio of about 1:1. In another variant the disaccharide alcohol composition of the substrate comprises 1,6-GPS from 53 to 99 % by weight and 1,1-GPM from 47 to 1 % by weight, respectively. The disaccharide alcohol composition of the substrate may further comprise 1,1-GPS.

The disaccharide alcohol or disaccharide alcohol composition is preferably agglomerated to form a sugar-free starter pellet or tablet which is to be coated according to the present invention. The disaccharide alcohol or disaccharide alcohol composition preferably has a particle size distribution in the range of about 680-830-940 (d10-d50-d90-quantiles, as measured by mechanical sieve shaker). Such a variant is known, for example, as galenIQ® 980 (Palatinit, DE).

### API

In this invention, the terms "API", "active principle", "compound", or "drug" refer to substances or agents that are mainly used for the treatment, prevention and diagnosis of diseases or impaired conditions of humans and animals. Specifically, they include anti-epileptic agents (e.g. phenytoin, acetylpheneturide, trimethadione, phenobarbital, primidone etc.), antipyretic-analgesic-anti-inflammatory agents (e.g. acetaminophen, phenyl acetylglycine methyl amide, mefenamic acid, diclofenac sodium, floctafenine, aspirin, aspirin aluminum, ethenzamide, oxyphenbutazone, sulpyrin, phenylbutazone, ibuprofen, alclofenac, naproxen, ketoprofen, tinoridine hydrochloride, benzydamine hydrochloride, tialamide hydrochloride, indomethacin, piroxicam, salicylamide etc.), antivertigo agents (e.g. dimenhydrinate, meclizine hydrochloride, diphentoin hydrochloride etc.), narcotics (e.g. opium alkaloid hydrochlorides, ethylmorphine hydrochloride, codeine phosphate, dihydrocodeine phosphate, oxymethebanol etc.), agents for psychological use (e.g. chlorpromazine hydrochloride, levomepromazine maleate, perazine maleate, propericiazine, perphenazine, chlorprothixene, haloperidol, diazepam, oxazepam. oxazolam, mexazolam, alprazolam, zotepine, methylphenidate, amphetamine etc.), skeletal muscle relaxants (e.g. chlorzoxazone, chlorphenesin carbamate, chlormezanone, pridinol mesylate, eperisone hydrochloride etc.), autonomic nerve agents (e.g. betanecol hydrochloride, neostigmine bromide, pyridostigmine bromide etc.), antispasmodic agents (e.g. atropine sulfate, butropium bromide, butylscopolammonium bromide, propantheline bromide, papaverine hydrochloride etc.), antiparkinsonian agents (e.g. biperiden hydrochloride, trihexyphenidyl hydrochloride, amantadine hydrochloride, levodopa etc.), ophthalmological agents (e.g. dichlorophenamide, metazolamide etc.), antihistaminic agents (e.g. diphenhydramine hydrochloride, di-chlorpheniramine maleate, d-chlorpheniramine maleate, promethazine, mequitazine, clemastine fumarate etc.), cardiotonics (e.g. aminophylline, caffeine, dl-isoproterenol hydrochloride, etilefrin hydrochloride, norfenefrine hydrochloride, ubide-carenone etc.), antiarrhythmic agents (e.g. procainamide hydrochloride, pindolol, metoprolol tartrate, disopyramide etc.), diuretics (e.g. potassium chloride, cyclopenthiazide, hydrochlorothiazide, triamterene, furosemide etc.), antihypertensive agents (e.g. hexamethonium bromide, hydralazine hydrochloride, syrosingopine, reserpine, propranolol hydrochloride, captopril, methyidopa etc.), vasoconstrictors (e.g. dihydroergotamine mesylate etc.), vasodilators (e.g. etafenone hydrochloride, diltiazem hydrochloride, carbochromen hydrochloride, pentaerythritol tetranitrate, dipyridamole, isosorbide nitrate, nifedipine, nicametate citrate, cyclandelate, cinnarizine etc.), agents for arteriosclerosis (e.g. ethyl linoleate, lecithin, clofibrate etc.), agents for the circulatory system (e.g. nicardipine hydrochloride, meclofenoxate hydrochloride, cytochrome C, pyridinol carbamate, vinpocetine, hopantenate calcium, pentoxifylline, idebenone etc.), respiratory stimulants (e.g. dimefline hydrochloride etc.), antitussives and expectorants (e.g. codeine phosphate, dihydrocodeine phosphate, dextromethorphan hydrobromide, noscapine, L-cysteine methyl ester hydrochloride, bromhexine hydrochloride, theophylline, ephedrine hydrochloride, amlexanox etc.), hepatoprotectants (e.g. osalmid, phenyl propanol, hymecromone etc.), agents for intestinal disorders (e.g. berberine hydrochloride, loperamide hydrochloride etc.), agents for digestive organs (e.g. metoclopramide, fenipentol, domperidone etc.), vitamins (e.g. retinol acetate, dihydrotachysterol, etretinate, thiamine hydrochloride, thiamine sulfate, fursultiamine, riboflavin, pyridoxine hydrochloride, pyridoxal phosphate, nicotinic acid, pantethine, cyanocobalamin, biotin, ascorbic acid, phytonadione, menatetrenome etc.), antibiotics (e.g benzathine benzylpenicillin, amoxicillin, ampicillin, cyclacillin, cefaclor, cephalexin, erythromycin, kitasamycin, josamycin, chloramphenicol, tetracycline, griseofulvin, cefuzonam etc.), chemical therapeutic agents (e.g sulfamethoxazole, isoniazid, ethionamide, thiazosulfone, nitrofurantoin, enoxacin, ofloxacin, norfloxacin etc.).

Embodiments of the invention thus also include controlled release drug formulations comprising one or more of the above API. The medicaments are present in pharmaceutically effective amounts. It is preferred that the API is present in amounts ranging from about 0.5% to about 90% by dry weight of the unit dosage form. The API may be associated with a pharmaceutical carrier in the core. These include lubricants, excipients, such as plasticizers and fillers, and the like.

In this invention "API" or "active pharmaceutical principle" or "drug" also refer to substances that are mainy used to condition, enhance or modify nutritional preparations and compositions, e.g. novel food products or nutriceuticals. Specific examples are aroma formulations, vitamins or other excipients, e.g. antioxidants preservatives, colors etc., which require a controlled or controllable release profile in the food product. In particular either during storage or preparation of the food product, or during or after consumption.

"API" or "active pharmaceutical principle" or "drug" also refer to substances that are mainy used to condition, enhance or modify cosmetic preparations. Specific examples are antioxidants cosmetically active formulations or other excipients, e.g. preservatives, colors etc., which require a controlled or controllable release profile in the cosmetic product.

In addition to medicinal or cosmetic uses, the products of the invention can also be used for agricultural chemicals, fertilizers, seeds or other industrial applications where a controlled or controllable release profile is required.

### Drug layering

In a preferred embodiment the active ingredient (API) is present in a solution or suspension and is layered on an inert substrate. The drug-layered core is the coated with the coating composition of the invention on starter pellets, e. g., a substrate which is preferably a sphere, bead or seed. The starter particles or seeds can be any free flowing non-friable granular material such as sucrose, Isomalt, lactose or MCC particularly as described hereinabove. Preferably, it is a sphere having an average diameter of from about 0.5 mm to about 1.5 mm.

Drug layering of the starter pellets/substrates in accomplished utilizing methods are known in the art. Preferred coating/layering processes are described herein after in connection with "coating". The following parameters for the drug layering process are preferred: inlet air temperature: approx. 59° C; outlet air temperature: approx. 51 to 53° C; spray rate approx. 2 to 3 mL/min; spray pressure: approx. 0.8 bar; spray mode: bottom spray.

The drug layer is preferably applied in an amount of 5 % to 50 % by weight of the inert substrate dry weight.

The drug layering compositions comprise the API, at least one solvent or solvent composition, and preferably at least one binder or binder composition. A binder promotes adhesion of the drug to the beads and is present in binding effective amounts. Preferably, the binding agent is present in amounts of from about 0.1 to about 45% by weight of the core element and more preferably from about 0.1 to about 20% by weight and most preferably approximately about 3 to about 15% by weight, based on the total weight of the drug layer.

The binding agent may be any suitable type used in the pharma ceutical arts. Suitable binders are selected from polyvinyl-pyrrolidine, hydroxypropyl cellulose, hydroxypropyl methyl cellulose (HPMC), sugars (e.g. glucose), acacia, carboxymethylcellulose sodium, dextrin, ethylcellulose, gelatin, pregelatinized starch, sodium aldinate, zein and the like. A preferred binder for drug layering is HPMC. The binding agent may be provided in the form of a granulating solution.

An aqueous or organic solvent may be included. Methanol, ethanol or mixtures thereof may be used as solvents.

Besides the API and a binder that promotes adhesion of the drug to the starter seeds, the core may also contain antiadherents that prevent or minimize agglomeration during the layering process, and other ingredients such as those described hereinabove.

### Coating process

The invention includes a process for coating a substrate with a preferably fully aqueous latex or pseudolatex film coating composition as described hereinabove. The coating step may be followed by a heat treatment step. The coating step may optionally be carried out under high relative humidity conditions and the heating step may be carried out under low relative humidity conditions. The heat treatment step is preferably carried out at a temperature above a minimum film-forming temperature of the coating composition.

The coating composition of the present invention is coated onto the core carrying or containing the API in any conventional oral unit dosage form, such as a tablet, capsule, pill, granule or powder to form the desired preparation. The coating composition of the present invention coats the central core element preferably by utilizing conventional methods known in the art. For example, the coating composition of the present invention coats the central core in a fluidized bed or pan. Other preferred examples include spraying or painting the suspension of the composition of the present invention onto the formulation, including immersing the core element in suspension of the coating composition of the present invention. Most preferably, the coating composition of the invention is applied to the core element, e. g., the drug pellets, in a fluid bed bottom spray coater by having the pellets suspended in an air stream, and an aqueous dispersion of the coating composition is sprayed thereon.

Various conventional coating apparatuses may be employed to facilitate this, including, for example, a centrifugal fluidized bed coating apparatus, a pan coating apparatus, or a fluidized bed granulating coating apparatus. In the processes described herein, it is to be understood that during the coating of the core and/or after the core is completely coated, the solvent, i. e., water is removed by techniques known to one of ordinary skill in the art such as by drying or curing, and the like. As used herein, the terms "coating" or "coat" or synonyms thereto includes both the process of applying the coating composition of the present invention to the core and the concomitant removal of the solvent, e. g., water, by techniques known to one of ordinary skill in the art, e. g., drying or curing thereof in which substantially all of the solvent e. g., water, in the coating composition is removed.

The coating process is most preferably based on the concept of "Wurster coating", also known as "bottom spray" or "air suspension coating". According to this, particles are suspended in an air or gas stream and the coating formulation is sprayed onto the particles while they are suspended. In the fluidised bed coater the coating composition is sprayed from the bottom through at least one nozzle. In Wurster-type coating an inner area of high velocity is produced that separates the particles and pneumatically transports them past the spray nozzle. The coating dries while the particles are suspended to prevent agglomeration in a low velocity expanded area of the chamber. During the coating the coated particles are heated to a certain temperature dependent on the polymer where the polymer film is to be formed.

According to the invention, the following coating parameters are preferred for Wurster-type coating: inlet air temperature: approximately 48 to 51° C; outlet air temperature: approximately 37 to 43° C; spray rate: approx. 3 to 5 mL/min; spray pressure: approx. 0.8 bar.

The amount of coating applied is sufficient to retard the release of the active component at a desired state. By varying the proportion of the coating on the core, different release profiles of the API are obtained. The coating composition is applied to the core in a thickness sufficient to obtain the desired release profile of a therapeutically active agent when the coated substrate is exposed to aqueous media like Saliva or gut juices. Thus, by decreasing or increasing the thickness of the core, and by raising or lowering the amount of release controlling agent, i.e. disaccharide alcohol or composition, the dissolution profile is varied. Preferably, the coating composition is applied to the core at a thickness ranging from about 1 % to about 15% by dry weight of the composition and more preferably from about 2% to about 10.

In this invention, the thickness of the film of film coated particles should generally be, but not limited to, 30 µm or more. When the film becomes thicker than 100 µm, there is the effect that the release rate may be excessively slowed. The quantity of coating varies greatly depending on the area, particle size and shape of the uncoated particles and the smoothness of their surfaces. However, though not limiting, it should be on the order of 1 to 100 parts by weight, and, preferably, on the order of 3 to 25 parts by weight, per 100 parts by weight of the uncoated particles.

The examples and figures are given to illustrate the invention, but the invention is not restricted thereto.

### Example: Controlled release formulation comprising diclofenac-Na

To produce a controlled release formulation, comprising diclofenac sodium salt (diclofenac-Na) as the active principle (API). In the particulate formulation inert cores are layered with the API, the drug-layered spheres are then coated with a film-forming coating composition.

As inner core starter pellets of microcrystalline cellulose (MCC) are used. The starter pellets have diameters of approximately 700 to 1000 µm. As preferred example Ethispheres® 850 (mesh 18/25) (NP Pharm, FR) is used: At least 96 % lie in the size range of 710 to 1000 µm. Degree of polymerization is 350 at most. The pH of the neutral inert pellets lies in the range of 5.0 to 7.5.

A layering composition is provided, comprising diclofenac-Na, hydroxypolymethyl cellulose (HPMC) as binder, sunset yellow TM or E 110 as coloring agents, and water as solvent. The inert starter pellets are transferred to a fluid bed coating apparatus with bottom spray unit, for example, Wurster, Glatt with Wurster unit, Hüttlin Kugel-coater, Aeromatic or similar devices. For Aeromatic STREA-1 the following parameters are used: Batch size: 450 g, air flow: 80 m³/h, spray pressure: 0,8 bar (bottom-spray), spray rate: 2.4 mL/min, inlet air temperature: 59° C, outlet air temperature: 51° C to 53° C.

After drug layering, three aliquots (150 g each) are provided for coating. Coating compositions are based on methacrylic co-polymers with trimethyl-ammonioethyl methacrylate as film-forming polymers, in particular Eudragit® RS 30 D (Röhm Pharma, DE). The coating compositions further comprise talc as antisticking agent and triethyl citrate (TEC) as plasticizer.

### Coating compositions (% by weight of the suspension)

Batch 1 (Control): Eudragit® RS 30 D: 35-50 %

| | |
|---|---|
| Talc: | 4-10 % |
| TEC: | 1-3 % |
| Water: | 40-60 % |

Batch 2 (invention): see Batch 1 + galenIQ 721: 20 % of polymer dry weight

Batch 3 (invention): see Batch 1 + galenIQ 721: 40 % of polymer dry weight

The batches of coating dispersions are adjusted to the same solid content (approx. 20 %).

Coating is performed in a fluid bed coating apparatus with bottom spray unit (see above). For Aeromatic STREA-1 the following parameters apply: Batch size: 150 g, air flow: 80 m³/h, spray pressure: 0.8 bar (bottom-spray), spray rate: 3 to 5 mL/min, inlet air temperature: 48° C to 51° C, outlet air temperature: 37° C to 43° C. The coating is continued to a coating level of 5 %. In a second approach the coating of the batches is continued to a coating level of 10 %.

Dissolution profiles of each batch of coated particles (150 g) are assessed according to U.S.P. No. 1 apparatus (basket). For example, a Hanson SR8 dissolution test station is utilized. The following parameters apply: volume of dissolution: 900 mL, rotation speed: 100 rpm, temperature: 37° C, buffer (phosphate): either pH 6.8 or 4.5.

Results of the dissolution tests are illustrated in Figures 1A to 1D.

## Claims

**1.** A controlled release coating composition for particulate drug formulations, comprising:
- film-forming component comprising polymeric binder; and
- release controlling agent, selected from disaccharide alcohols and disaccharide alcohol compositions.

**2.** The coating composition of claim 1, wherein the coating composition comprises disaccharide alcohol or disaccharide alcohol composition from 1 to 20 % by weight dry matter of the coating composition.

**3.** The coating composition of claim 1 or 2, wherein the coating composition comprises disaccharide alcohol or disaccharide alcohol composition from 5 to 50 % by weight dry matter of the film-forming component.

**4.** The coating composition of any one of claims 1 to 3, wherein the disaccharide alcohol composition comprises 1,6-GPS from 1 to 99 % by weight and 1,1-GPM from 99 to 1 % by weight.

**5.** The coating composition of claim 4, wherein the disaccharide alcohol composition further comprises 1,1-GPS.

**6.** The coating composition of claim 4 or 5, wherein the disaccharide alcohol composition comprises 1,6-GPS from more than 47 to less than 53 % by weight and 1,1-GPM from less than 53 to more than 47 % by weight, respectively.

**7.** The coating composition of claim 4 or 5, wherein the disaccharide alcohol composition comprises 1,6-GPS from 53 to 99 % by weight and 1,1-GPM from 47 to 1 % by weight, respectively.

**8.** The coating composition of any one of claim 1 to 3, wherein the disaccharide alcohol composition is maltitol.

**9.** The coating composition of any one of claim 1 to 3, wherein the disaccharide alcohol composition is lactitol.

**10.** The coating composition of any one of the preceding claims, which is sugar-free.

**11.** The coating composition of any one of the preceding claims, wherein the film-forming agent is a polymethacrylate-based polymer.

**12.** The coating composition of any one of the preceding claims, further comprising one or more of excipients: plasticizers and antisticking agents.

**13.** The coating composition of any one of the preceding claims, which comprises water as solvent and is free of organic solvent

**14.** The coating composition of any one of the preceding claims, which exhibits a pH-dependent release.

**15.** The coating composition of claim 14, which is an enteric coating.

**16.** A particulate drug formulation, comprising the controlled release coating of any one of claims 1 to 15.

**16.** The particulate drug formulation of claim 15, comprising an inert inner core, at least one intermediate drug layer containing drug or a drug composition, and the controlled release coating of any one of claims 1 to 15 as outer coating.

**17.** The particulate drug formulation of claim 16, wherein the inert core is sugar-free.

**18.** The particulate drug formulation of claim 16 or 17, wherein the inert core comprises microcrystalline cellulose.

**19.** The particulate drug formulation of any one of claim 16 to 18, wherein the intermediate drug layer further comprises one or more of excipients: binding agents and bulking agents.

**20.** The particulate drug formulation of claim 16, comprising an inner core containing drug or a drug composition and the controlled release coating of any one of claims 1 to 15 as an outer coating.

**21.** The particulate drug formulation of claim 20, wherein the drug containing inner core further comprises one or more of excipients: binding agents and bulking agents.

**22.** The particulate drug formulation of any one of claim 16 to 21, wherein the drug or drug composition exhibits a solubility in water (at 25°C) of 50 mg/mL or less.

**23.** The particulate drug formulation of claim 22, wherein the drug or drug composition exhibits a solubility in water (at 25°C) of 10 mg/mL or less.

**24.** A multiparticulate pharmaceutical, nutritional or cosmetic formulation, comprising a multitude of the particulate drug formulation of any one of claim 16 to 23.

**25.** The multiparticulate pharmaceutical, nutritional or cosmetic formulation of claim 24 in the form of a capsule.

**26.** The multiparticulate pharmaceutical, nutritional or cosmetic formulation of claim 24 in the form of a compacted tablet.

**27.** A method for producing a particulate drug formulation with controlled release property, comprising:
providing a starter pellet as inert inner core;
layering the starter pellet with a layer comprising drug or a drug composition; and
coating the layered pellet with the coating composition of any one of claim 1 to 15;

**28.** Use of disaccharide alcohol or disaccharide alcohol composition as release controlling agent in a controlled release coating composition for particulate drug formulations.
